# EUROPEAN PATENT APPLICATION

(11) **EP 2 078 748 A1**
(43) Date of publication of application: **15.07.2009**
(21) Application number: 06810829.9
(22) Date of filing: 29.09.2006
(51) Int. Cl.: C12N 15/09, C12N 9/88, C12P 13/00

(54) **NOVEL HIGHLY ACTIVE MODIFIED TYPE S-HYDROXYNITRILE LYASE**

(71) Applicant: Nippon Shokubai Co., Ltd., Chuo-ku, Osaka-shi Osaka 541-0043 (JP)
(72) Inventor: SEMBA, Hisashi, Tsuchiura-shi, Ibaraki 300-0810 (JP); ICHIGE, Eita, Chikusei-shi, Ibaraki 308-0847 (JP)
(74) Representative: Williams, Aylsa
(86) International application number: PCT/JP2006/319423
(87) International publication number: WO 2008/041272

(57) **Abstract**

This invention relates to a novel high-activity modified S-hydroxynitrile lyase (SHNL). More particularly, this invention relates to novel high-activity modified SHNL that is obtained by substituting amino acids at given sites (14, 44, 66, 94, 103, 118, 122, 125, 127, 129, 147, 148, 152, 212, and 216) or inserting amino acid at a given site (between amino acids 128 and 129) of the amino acid sequence (SEQ ID NO: 2) of wild-type SHNL.

## Description

### Technical Field

The present invention relates to a novel high-activity modified S-hydroxynitrile lyase (SHNL). More particularly, the present invention relates to SHNL that is obtained by modifying an amino acid sequence at a specific site and has improved activity compared with wild-type SHNLs.

### Background Art

S-Hydroxynitrile lyase (SHNL) catalyzes the reaction between hydrocyanic acid and aldehyde or ketone to generate optically active cyanohydrins. Optically active cyanohydrins are important intermediates for synthesizing medicines. Accordingly, it can be said that SHNL is an industrially important enzyme.

SHNLs derived from Cassava *(Manihot esculenta*), Pará rubber tree (*Hevea brasiliensis*), poaceous plants (i.e., sorghum (*Sorghum bicolor*)), and the like have been known. At the industrial level, recombinant SHNL is preferably used in addition to wild-type SHNLs because separation of enzymes from organisms incurs a high cost.

Recombinant SHNL can be produced using *E. coli,* yeast, or other hosts. From an industrial point of view, use of modified SHNL with improved activity is preferable in order to further improve cost performance.

In order to enhance enzyme activity, a modified SHNL in which tryptophan 128 of the SHNL amino acid sequence had been substituted with alanine has been developed (JP Patent Publication (kokai) No. 2000-125886 (A); Lauble et al., Protein Science, 2002, 11: pp. 65-71). However, mutation of SHNL for the purpose of enhancing activity has not yet been reported, except for amino acid 128 of the amino acid sequence.

### Disclosure of the Invention

An object of the present invention is to provide novel SHNL with significantly improved activity compared with wild-type SHNL.

The present inventors have conducted concentrated studies in order to attain the above object. As a result, they found that amino acid substitution in SHNL through genetic engineering could produce enzymes having significantly improved activity compared with the enzyme before modification. This has led to the completion of the present invention.

More specifically, the present invention provides modified SHNL, which is obtained by substituting at least one amino acid selected from amino acids 14, 44, 66, 94, 103, 118, 122, 125, 127, 129, 147, 148, 152, 212, and 216 in the amino acid sequence (SEQ ID NO: 2) of wild-type SHNL derived from cassava (*Manihot esculenta*) with another amino acid, modified SHNL, which is obtained by substituting at least one amino acid selected from amino acids 14, 44, 66, 94, 103, 118, 122, 125, 127, 129, 146, 147, 151, 211, and 215 in the amino acid sequence (SEQ ID NO: 4) of wild-type SHNL derived from the Pará rubber tree (*Hevea brasiliensis*) with another amino acid, or modified SHNL, which is obtained by inserting at least one amino acid between amino acids 128 and 129 of the amino acid sequence of the cassava- or Pará rubber tree-derived wild-type SHNL.

Specifically, the present invention provides modified SHNL comprising at least one amino acid substitution selected from among the substitutions shown below in the amino acid sequence of wild-type SHNL derived from cassava (*Manihot esculenta*):
a) substitution of histidine 14 with glutamine;
b) substitution of aspartic acid 44 with asparagine;
c) substitution of glutamic acid 66 with aspartic acid or lysine;
d) substitution of valine 94 with alanine, cysteine, histidine, lysine, phenylalanine, proline, serine, threonine, tryptophan, or tyrosine;
e) substitution of histidine 103 with glutamine;
f) substitution of valine 118 with leucine;
g) substitution of leucine 122 with isoleucine, valine, glycine, or serine;
h) substitution of phenylalanine 125 with asparagine, cysteine, histidine, glutamine, arginine, leucine, isoleucine, threonine, or proline;
i) substitution of aspartic acid 127 with glutamic acid, asparagine, or glycine;
j) substitution of arginine 129 with histidine;
k) substitution of methionine 147 with threonine, serine, tyrosine, or cysteine;
l) substitution of lysine 148 with asparagine;
m) substitution of valine 152 with alanine or glycine;
n) substitution of leucine 212 with proline; and
o) substitution of glutamine 216 with histidine; or
modified SHNL, which is obtained by inserting glycine or lysine between amino acids 128 and 129 of the amino acid sequence of the cassava- or Pará rubber tree-derived wild-type SHNL.

The aforementioned modified SHNL may further comprise substitution of glycine 165 with glutamic acid and substitution of valine 173 with leucine.

For example, modified SHNL that comprises at least one amino acid substitution selected from:
d) substitution of valine 94 with alanine, cysteine, histidine, lysine, phenylalanine, proline, serine, threonine, tryptophan, or tyrosine;
g) substitution of leucine 122 with isoleucine, valine, glycine, or serine;
h) substitution of phenylalanine 125 with asparagine, cysteine, histidine, glutamine, arginine, leucine, isoleucine, threonine, or proline; and
o) substitution of glutamine 216 with histidine, in addition to substitution of glycine 165 with glutamic acid and substitution of valine 173 with leucine in the amino acid sequence of the cassava-derived wild-type SHNL, has a high level of enzyme activity.

In particular, modified SHNL that comprises substitution of phenylalanine 125 with asparagine and substitution of leucine 122 with serine in the amino acid sequence of the cassava-derived wild-type SHNL or modified SHNL that comprises substitution of glutamine 216 with histidine, in addition to the 2 aforementioned substitutions, has a remarkably high level of enzyme activity.

The present invention also provides DNA encoding the amino acid sequence of the aforementioned modified SHNL.

The modified SHNL of the present invention can be easily produced by introducing the DNA into an adequate host, culturing the same therein, and recovering a protein having SHNL activity from the resulting culture product.

The resulting modified SHNL has higher enzyme activity than that of wild-type SHNL, and it can produce optically active cyanohydrin of high purity from a carbonyl compound and cyanide. The present invention also provides a method for producing such modified SHNL and a method for producing optically active cyanohydrin using the same.

The high-activity modified SHNL of the present invention has remarkably improved enzyme activity compared with the enzyme activity of conventional enzymes. Accordingly, industrial production of optically active cyanohydrin can be enhanced.

### Brief Description of the Drawings

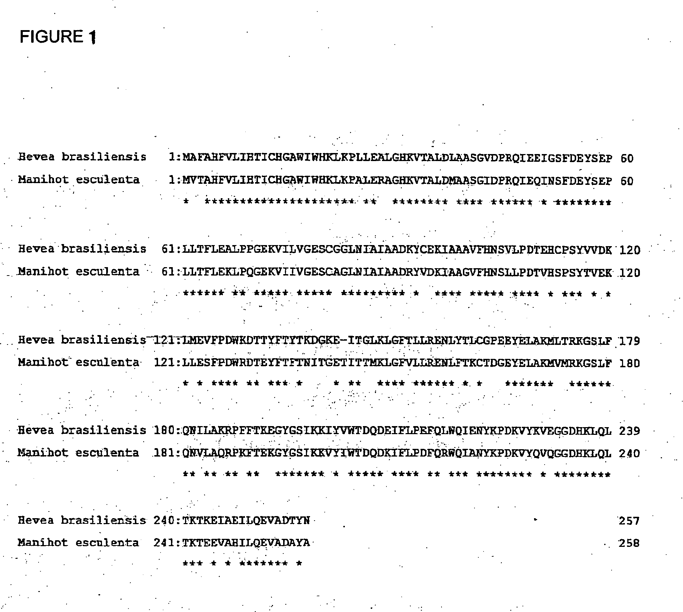

Fig. 1 is an alignment diagram showing amino acid sequences of SHNL derived from cassava (*Manihot esculenta*) and SHNL derived from the Pará rubber tree (*Hevea brasiliensis*).

### Best Modes for Carrying out the Invention

Hereafter, the present invention is described in detail.

### 1. Wild-type S-hydroxynitrile lyase

In the present invention, the term "wild-type S-hydroxynitrile lyase (hereafter abbreviated to "SHNL")" refers to SHNL isolated and purified from a plant or SHNL having an amino acid sequence identical to that of the former SHNL. The origin of wild-type SHNL is not particularly limited. Examples thereof include SHNL derived from poaceous plants such as sorghum (*Sorghum bicolor*), SHNL derived from Euphorbiaceae plants such as cassava (*Manihot esculenta*) or Pará rubber tree (*Hevea brasiliensis*), and SHNL derived from Olacaceae plants such as *Ximenia americana*. The amino acid sequences and the nucleotide sequences of the genes of such SHNLs are already known and can be easily obtained from public databases such as GenBank. For example, the SHNL gene derived from the Pará rubber tree (*Hevea brasiliensis*), the SHNL gene derived from *Manihot esculenta*, and the SHNL gene derived from sorghum are registered in GenBank under the accession nos. U40402 (SEQ ID NO: 3 is equivalent to CDS of U40402), Z29091, and AJ421152, respectively.

Fig. 1 shows the aligned amino acid sequences of the cassava (*Manihot esculenta*)-derived SHNL and of the Pará rubber tree (*Hevea brasiliensis*)-derived SHNL. Amino acid homology between these SHNLs is 74%, and amino acids of each SHNL are not completely identical. For example, *Hevea brasiliensis*-derived SHNL lacks the amino acid that is equivalent to amino acid 139 of *Manihot esculenta*-derived SHNL. Thus, the amino acid number in the helix D3' domain is out of alignment by one residue. Specifically, amino acids 14, 44, 66, 94, 103, 118, 122, 125, 127, 128, 129, 147, 148, 152, 212, and 216 in the amino acid sequence (SEQ ID NO: 2) of SHNL derived from cassava (*Manihot esculenta*) are equivalent to amino acids 14, 44, 66, 94, 103, 118, 122, 125, 127, 128, 129, 146, 147, 151, 211, and 215 in the amino acid sequence (SEQ ID NO: 4) of the Pará rubber tree (*Hevea brasiliensis*)-derived SHNL. SHNLs derived from cassava and derived from the Pará rubber tree belong to the α/β hydrolase superfamily and their conformations are very similar to each other. Based on the effects of modification of an amino acid sequence by SHNL derived from cassava, accordingly, similar effects can be expected concerning SHNL derived from the Pará rubber tree (*Hevea brasiliensis*) by modification of an amino acid sequence at a relevant site.

### 2. Modified S-hydroxynitrile lyase

The present invention relates to high-activity modified SHNL, which is obtained by modification (i.e., substitution or insertion) of an amino acid sequence at a given site of an amino acid sequence of wild-type SHNL derived from cassava or from Pará rubber tree. Specifically, the present invention relates to modified SHNL, which is obtained by substituting at least one amino acid selected from amino acids 14, 44, 66, 94, 103, 118, 122, 125, 127, 129, 147, 148, 152, 212, and 216 in the amino acid sequence (SEQ ID NO: 2) of wild-type SHNL derived from cassava (*Manihot esculenta*) with another amino acid, modified SHNL, which is obtained by substituting at least one amino acid selected from amino acids 14, 44, 66, 94, 103, 118, 122, 125, 127, 129, 146, 147, 151, 211, and 215 in the amino acid sequence (SEQ ID NO: 4) of wild-type SHNL derived from the Pará rubber tree (*Hevea brasiliensis*) with another amino acid, and modified SHNL, which is obtained by inserting at least one amino acid between amino acids 128 and 129 of the amino acid sequence of the cassava- or Pará rubber tree-derived wild-type SHNL (SEQ ID NO: 2 or 4).

A more specific example of modified SHNL is one comprising at least one amino acid substitution selected from the amino acid substitutions shown below in the amino acid sequence (SEQ ID NO: 2):
a) substitution of histidine 14 with glutamine;
b) substitution of aspartic acid 44 with asparagine;
c) substitution of glutamic acid 66 with aspartic acid, lysine, or arginine;
d) substitution of valine 94 with alanine, cysteine, histidine, lysine, phenylalanine, proline, serine, threonine, tryptophan, or tyrosine;
e) substitution of histidine 103 with glutamine;
f) substitution of histidine 118 with glutamine;
g) substitution of leucine 122 with isoleucine, valine, glycine, or serine;
h) substitution of phenylalanine 125 with asparagine, cysteine, histidine, glutamine, arginine, leucine, isoleucine, threonine, or proline;
i) substitution of aspartic acid 127 with glutamic acid, asparagine, or glycine;
j) substitution of arginine 129 with histidine;
k) substitution of methionine 147 with threonine, serine, tyrosine, or cysteine;
l) substitution of lysine 148 with asparagine;
m) substitution of valine 152 with alanine or glycine;
n) substitution of leucine 212 with proline; and
o) substitution of glutamine 216 with histidine; or
modified SHNL, which is obtained by inserting glycine or lysine between amino acids 128 and 129 of the amino acid sequence (SEQ ID NO: 2 or 4) of the cassava- or Pará rubber tree-derived wild-type SHNL.

Further, SHNL comprising combination of the aforementioned mutation sites has higher activity. For example, modified SHNL comprising amino acid substitutions at positions 94, 122, 125, and 216 in the amino acid sequence (SEQ ID NO: 2) of wild-type SHNL derived from cassava has high enzyme activity. Among them, modified SHNL comprising substitution of phenylalanine 125 with asparagine, that of leucine 122 with serine, or that of glutamine 216 with histidine has remarkably high enzyme activity.

Amino acid substitution and insertion may be carried out via site-directed mutagenesis into a gene that encodes the amino acid sequence in accordance with a conventional technique. Such site-directed mutagenesis can be easily performed using a commercially available kit (for example, QuikChange XL Site-Directed Mutagenesis Kit (Stratagene) or Transformer^{™} Site-Directed Mutagenesis Kit (Clontech.)). Since the modified SHNL of the present invention has enzyme activity higher than from that of wild-type SHNL, the modified SHNL of the present invention can be a very useful enzyme for industrial production of optically active cyanohydrin.

### 3. Production of high-activity modified S-hydroxynitrile lyase

### 3.1 DNA that encodes high-activity modified SHNL

DNA that encodes the modified SHNL protein according to the present invention is obtained by introducing site-directed mutations into the gene of known wild-type SHNL. Specifically, a pair of primers that can modify a codon at a substitution site into a codon that encodes the amino acid of interest is designed. Next, the resulting pair of primers is used to perform an extension reaction utilizing DNA that encodes wild-type SHNL as a template. Site-directed mutagenesis can be easily carried out utilizing commercialized kits (for example, QuikChange XL Site-Directed Mutagenesis Kit (Stratagene) or Transformer^{™} Site-Directed Mutagenesis Kit (Clontech)).

### 3.2 Recombinant vector

DNA that encodes the aforementioned high-activity modified SHNL is then ligated (inserted) to (into) a known vector such as a plasmid to prepare a recombinant vector. Such vector is not particularly limited as long as it can replicate the gene of interest in a host. Examples include plasmid DNA and phage DNA.

Examples of such plasmid DNA include E. coli-derived plasmids (for example, a pET21 vector having a particularly potent T7 promoter, such as pBR322, pBR325, pUC18, pUC119, pHCEIIB, pTrcHis, or pBlueBacHis, is preferable), *Bacillus subtilis-derived* plasmids (for example, pUB110 and pTP5), and yeast-derived plasmids (for example, YEp13, YEp24, YCp50, and pYE52). An example of phage DNA is λphage.

The gene of the present invention is inserted into the aforementioned vector by first cleaving purified DNA with an adequate restriction enzyme and inserting the cleaved DNA into an adequate restriction enzyme site or multi-cloning site of the vector DNA for ligation.

In order to express a foreign gene in a host, an adequate promoter needs to be positioned prior to the positioning of a structural gene. Such promoter is not particularly limited. Any promoter that is known to function in a host can be employed. Promoters will be described in detail concerning each host in sections concerning transformants below. If necessary, a cis element such as an enhancer, splicing signal, poly A additional signal, libosome binding sequence (SD sequence), terminator sequence, and the like may be positioned.

### 3.3 Modified SHNL expression system (transformant)

Subsequently, the aforementioned recombinant vector is introduced into a host in a manner such that the target gene can be expressed therein to prepare a modified SHNL expression system. A host cell is not particularly limited as long as the DNA of the present invention can be expressed therein. Examples thereof include: bacteria belonging to *Escherichia* such as *Escherichia coli, Bacillus* such as *Bacillus subtilis, Pseudomonas* such as *Pseudomonas putida*, or *Rhizobium* such as *Rhizobium meliloti*; yeast such as *Saccharomyces cervisiae, Schizosaccharomyces pombe*, and *Pichia pastoris*; animal cells such as COS cells and CHO cells; and insect cells such as Sf19 and Sf21.

When a bacterium such as *E*. *coli* is used as a host, it is preferable that the recombinant vector of the present invention be capable of self-replicating in the bacterium and, at the same time, also be comprised of a promoter, a ribosome binding sequence, the gene of the present invention, and a transcription termination sequence. Further, it may also comprise a gene for regulating a promoter. Examples of *E*. *coli* include *Escherichia coli* HMS174 (DE3), K12, DH1, and B strains, and examples of *Bacillus subtilis* include *Bacillus subtilis* MI 114 and 207-21. A promoter is not particularly limited as long as it can express the gene of interest in a host such as *E. coli.* For example, *E*. *coli*-derived or phage-derived promoters can be employed, such as: trp promoter, lac promoter, P_{L} promoter, and P_{R} promoter. Alternatively, an artificially designed and modified promoter, such as a tac promoter, may also be employed. A method for introducing a recombinant vector into a bacterium is not particularly limited. For example, a method involving the use of calcium ions (Cohen, S. N. et al., Ploc. Natl. Acad. Sci., U.S.A., 69: 2110-2114, 1972) and electroporation can be employed.

Where yeast is used as a host, for example, *Saccharomyces cerevisiae*, *Schizosaccharomyces pombe*, or *Pichea pastris*, is used. A promoter is not particularly limited as long as it can express the gene of interest in yeast. For example, gall promoter, gal10 promoter, heat shock protein promoter, MFα1 promoter, PHO5 promoter, PGK promoter, GAP promoter, ADH promoter, or AOX1 promoter can be employed. A method for introducing a vector into yeast is not particularly limited. Examples of such methods include electroporation (Becker, D. M. et al., Methods. Enzymol., 194: 182-187, 1990), the spheroplast method (Hinnen, A. et al., Proc. Natl. Acad. Sci., U.S.A., 75: 1929-1933, 1978), and the lithium acetate method (Itoh, H., J. Bacteriol., 153: 163-168, 1983).

### 3.4 Culture of transformant

The modified SHNL of the present invention can be obtained by culturing the transformant according to the present invention in an adequate medium and recovering a protein having enzyme activity from the culture product. A method for culturing the transformant according to the present invention is adequately determined in accordance with the host. In the case of a transformant where a microorganism such as *E. coli* or yeast is employed as a host, for example, either a natural or synthetic medium may be used as long as it contains carbon sources, nitrogen sources, and inorganic salts assimilable by the microorganism and is capable of efficiently culturing the transformant.

During the culture, an antibiotic such as ampicillin or tetracycline may be added to the medium, if necessary. When a microorganism transformed with an expression vector containing an inducible promoter is cultured, an inducer may be added to the medium, if necessary. When a microorganism transformed with an expression vector containing lac promoter is cultured, for example, isopropyl-β-thiogalactopyranoside (IPTG) may be added to the medium. When culturing a microorganism transformed with an expression vector containing trp promoter, indoleacrylic acid (IAA) or the like may be added to the medium.

If the enzyme protein of the present invention is produced in the relevant microorganism or cell after the culture, the cultured microorganism or cell is homogenized. If the protein of the present invention is secreted outside of the microorganism or cell, the culture broth may be used in such state or may be subjected to centrifugation or another procedure to remove the microorganism or cell.

Ammonium sulfate precipitation, SDS-PAGE, gel filtration, ion exchange chromatography, affinity chromatography, or other means are employed independently or in an appropriate combination to isolate and purify proteins.

The enzyme activity of the modified SHNL according to the present invention can be confirmed by adding the enzyme to a reaction solution that contains a substrate, i.e., adequate cyanide, and aldehyde or ketone and detecting the generated optically active cyanohydrin. Optically active cyanohydrin can be confirmed by, for example, gas chromatography or high-performance liquid chromatography. Alternatively, an antibody that specifically binds to the modified SHNL of the present invention can be prepared to confirm the expression via Western blotting using the resulting antibody. For example, the enzyme activity of SHNL can be confirmed by assaying the amount of aldehyde generated per unit time (calculated based on absorbance at 249.6 nm) upon degradation of mandelonitrile by SHNL.

The modified SHNL of the present invention can be produced in accordance with the methods disclosed in JP Patent Publication (Kokai) Nos. 10-373246 A (1998), 10-373248 A (1998), or 11-367251 A (1999).

### 4. Synthesis of optically active cyanohydrin using high-activity modified SHNL

The modified SHNL of the present invention can synthesize optically active cyanohydrin with production efficiency and optical purity higher than those of wild-type SHNL. Optically active cyanohydrin can be synthesized with the use of the present invention's high-activity modified SHNL in the same manner as wild-type SHNL.

Specifically, the modified SHNL of the present invention and a reaction substrate are added to a reaction solvent, and the reaction is carried out at 10°C to 50°C for 20 minutes to 24 hours. Thus, optically active cyanohydrin can be synthesized. The reaction time is adequately determined in accordance with the conversion rate of the substrate. Examples of a reaction substrate that can be employed include a carbonyl compound and cyanide. A carbonyl compound is an aldehyde or ketone represented by COR1R2 wherein R1 and R2 each independently represent: a hydrogen atom, substituted or non-substituted, linear or branched, and saturated alkyl having 1 to 18 carbon atoms, or a substituted or non-substituted cyclic 5-22-membered aromatic group, provided that R1 and R2 do not simultaneously represent a hydrogen atom. Cyanide is not particularly limited as long as it generates cyanide ions (CN⁻). Examples thereof that can be employed include hydrogen cyanides such as sodium cyanide or potassium cyanide and cyanohydrins such as acetone cyanohydrin.

Use of a reaction solvent mainly composed of an organic solvent that is hardly soluble or insoluble in water is preferable from the viewpoint described below. That is, when a large quantity of water is present in a reaction system, racemization of optically active cyanohydrin generated via enzyme reaction is likely to occur. When aldehyde or ketone having a low degree of water solubility is used as a starting material, the production efficiency deteriorates. Such organic solvent is not particularly limited if it does not affect the synthesis of optically active cyanohydrin via enzyme reaction. An organic solvent can be adequately selected in accordance with properties of aldehyde or ketone used as a starting material for synthesis or properties of cyanohydrin, which is a generated product. Specific examples include: aliphatic or aromatic, linear, branched, or cyclic, and saturated or unsaturated hydrocarbon solvents that may be optionally halogenated, such as pentane, hexane, toluene, xylene, and methylene chloride; aliphatic or aromatic, linear, branched, or cyclic, and saturated or unsaturated alcohol solvents that may be optionally halogenated, such as isopropyl alcohol, n-butanol, isobutanol, t-butanol, hexanol, cyclohexanol, and n-amyl alcohol; aliphatic or aromatic, linear, branched, or cyclic, and saturated or unsaturated ether solvents that may be optionally halogenated, such as diethyl ether, dipropyl ether, diisopropyl ether, dibutyl ether, and methyl t-butyl ether; and aliphatic or aromatic, linear, branched, or cyclic, and saturated or unsaturated ester solvents that may be optionally halogenated, such as methyl formate, methyl acetate, ethyl acetate, butyl acetate, and methyl propionate. They may be used alone or in combinations of two or more. An aforementioned solvent that comprises or is saturated with water or an aqueous buffer can also be employed.

In the process of industrial production, the modified SHNL may be utilized as an enzyme immobilized on an adequate inorganic carrier (see, for example, JP Patent Publication (Kokai) No. 2002-176974 A). Examples of preferable methods for synthesizing cyanohydrin with the use of the modified SHNL of the present invention include those disclosed in JP Patent Publication (Kokai) Nos. 2002-355085 A, 2002-176974 A, 2001-363840 A, 2001-346596 A, 2001-190275 A, 2000-245286 A, 2001-120289 A, and 2000-217590 A.

### Examples

Hereafter, the present invention is described in greater detail with reference to examples, although the technical scope of the present invention is not limited thereto. Example 1: Screening for of high-activity mutant SHNL from library of random mutant SHNL

### [Material and method]

### 1) Library of random mutant SHNL

The SHNL gene used in the present invention comprised a sequence (SEQ ID NO: 1: JP Patent Application No. 2002-365675 (hereafter this SHNL gene is referred to as "SHNL-Wild") resulting from conversion of the gene sequence of SHNL cloned from cassava (*Manihot esculenta*) into an *E. coli*-type codon. With the use of such gene as a template, mutation was introduced randomly via Error prone PCR using the GeneMorph^{™}PCR Mutagenesis Kit (Stratagene). PCR was carried out using the primers having the sequences shown below.

Forward primer: 5'-GGG GGG GAA TTC ATG GTT ACT GCA CAC TTC GTT CTG ATT
CAC-3' (SEQ ID NO: 5)

Reverse primer: 5'-GGG GGG AAG CTT TTA AGC GTA TGC ATC AGC AAC TTC TTG
CAG-3' (SEQ ID NO: 6)

The resulting randomly-mutated SHNL gene was inserted into a multicloning site of a pKK223-3 vector plasmid (Amersham BioSciences) and transformed into *Escherichia coli* DH5α to prepare a library of random mutant SHNLs. The prepared library of random mutant SHNL was cryopreserved by the glycerol stock method.

### 2) Screening for of high-activity mutant enzyme

Clones obtained from the library of random mutant SHNL were inoculated on a deep-well plate into which NS-1 medium shown in Table 1 had been dispensed, and shake culture was carried out at 37°C and 900 rpm. When culturing clones obtained from the library of random mutant SHNL, several *E*. *coli* strains with recombinant SHNL-Wild genes were inoculated on the same plate as the control.

**[Table 1]**

| Composition of NS-1 medium | |
|---|---|
| Glycerol | 20 g |
| (NH₄)₂SO₄ | 5 g |
| KH₂PO₄ | 1 g |
| K₂HPO₄ | 3g |
| Yeast ext. (Ebios P2G) | 20 g |
| MgSO₄·7H₂O | 0.5 g |
| Ampicillin | 1 g |
| IPTG | 0.238 g |
| Distilled water | 1 liter |

After the cells were thoroughly grown, part of the culture solution was sampled using the screening robotic system (Beckman coulter), the culture solution was adequately diluted with distilled water, and the absorbance at 660 nm was assayed to determine the cell density. Similarly, 10 µl of the culture solution was applied to the deep-well plate comprising 300 µl each of the substrate solution dispensed in each well using the screening robotic system, and enzyme reaction was performed by shaking. Phosphoric acid (30 µl) was added 10 minutes later to terminate the reaction, and the reaction solution was separated from the cells via centrifugation. The separated reaction solution was transferred to a UV plate, the absorbance at 250 nm was assayed using a plate reader (GENios; TECAN), and the determined value was designated as the SHNL activity value. Based on this activity value, the activity value per unit cell density was determined. The obtained value was compared with that of *E*. *coli* strains of recombinant SHNL-Wild gene to select clones exhibiting an enhanced activity value per unit cell density.

The clones selected via the present screening were subjected to plasmid extraction. Using the resultant as a template, sequence analysis was carried out to identify the mutation site.

### [Results]

The results of evaluating the clones obtained from the library of random mutant SHNL selected by the screening robotic system are shown in Table 2. The results demonstrate that substitution of at least one amino acid selected from amino acids 14, 44, 66, 94, 103, 118, 125, 127, 129, 147, 148, 152, 212, and 216 of the SHNL amino acid (SEQ ID NO: 2) with another amino acid results in improvement in SHNL activity by 1.2 to 6.7 times.

**[Table 2]**

| Evaluation of clones obtained from the library of random mutant SHNL | | | | | | | |
|---|---|---|---|---|---|---|---|
| Strain No. | Amino acid No. | Nucleotide No. | Before mutation | | After mutation | | Activity value/ average of wild-type |
| 021A1 | 14 | 42 | His | CAC | Gln | CAA | 2.69 |
| 021 C4 | 44 | 130 | Asp | GAC | Asn | AAC | 3.09 |
| 025D3 | 66 | 198 | Glu | GAA | Asp | GAT | 5.17 |
| 020A4 | 66 | 196 | Glu | GAA | Lys | AAA | 2.82 |
| 011H7 | 94 | 281 | Val | GTT | Ala | GCT | 4.69 |
| 043G10 | 94 | 281 | Val | GTT | Ala | GCT | 2.03 |
| 048H6 | 94 | 281 | Val | GTT | Ala | GCT | 2.39 |
| 022B3 | 103 | 309 | His | CAC | Gln | CAG | 3.98 |
| 052G5 | 103 | 309 | His | CAC | Gln | CAA | 2.24 |
| 042H9 | 118 | 352 | Val | GTT | Leu | CTT | 1.30 |
| 050C12 | 125 | 374 | Phe | TTC | Ser | TCC | 1.55 |
| 028B3 | 127 | 380 | Asp | GAC | Glu | GAA | 5.08 |
| 005D6 | 127 | 380 | Asp | GAC | Gly | GGC | 1.27 |
| 030C7 | 127 | 379 | Asp | GAC | Asn | AAC | 6.27 |
| | 129 | 386 | Arg | CGT | His | CAT | |
| 002A7 | 127 | 380 | Asp | GAC | Gly | GGC | 1.50 |
| | 152 | 455 | Val | GTT | Ala | GCT | |
| 012F7 | 129 | 386 | Arg | CGT | His | CAT | 3.43 |
| | 152 | 455 | Val | GTT | Gly | GGT | |
| 020B10 | 129 | 386 | Arg | CGT | His | CAT | 2.77 |
| 039B4 | 147 | 439 | Met | ATG | Val | GTG | 1.41 |
| 012F2 | 148 | 444 | Lys | AAA | Asn | AAT | 2.88 |
| 012E10 | 212 | 635 | Leu | CTG | Pro | CCG | 3.83 |
| 018C12 | 216 | 648 | Gln | CAA | His | CAT | 6.70 |
| 005F6 | 128 | 383 | Trp | TGG | Leu | TTG | 1.79 |
| 033D10 | 128 | 384 | Trp | TGG | Cys | TGT | 2.03 |
| 046D9 | 128 | 383 | Trp. | TGG | Ser | TCG | 1.08 |

### Example 2: Substitution of amino acid 125 in SHNL amino acid sequence

Based on the experiment of Example 1, a high-activity mutant in which amino acid 125 of the amino acid sequence of Wild-SHNL (SEQ ID NO: 2), i.e., phenylalanine, had been substituted with tyrosine was obtained. Site-directed mutagenesis was applied to the amino acid 125 of the SHNL amino acid sequence to substitute the amino acid 125 with any of various types of amino acids. Thus, production of a high-activity mutant SHNL was further attempted.

### [Material and method]

Site-directed mutagenesis was performed using the QuikChange XL Site-Directed Mutagenesis Kit (Stratagene) to substitute the amino acid 125 with any of various types of amino acids. As a template, 10 ng of a vector plasmid, SHNL-G165E,V173L/pKK223-3, comprising the SHNL-G165E,V173L gene incorporated therein (i.e., the SHNL gene modified by substituting Gly 165 with Glu and Val 173 with Leu of the amino acid sequence of Wild-SHNL: shown in SEQ ID NO: 25, hereafter this gene is referred to as "Ver.0") was used (this plasmid is hereafter referred to as "Ver.0/pKK") (see Reference Examples). Primers shown below were used.

Forward primer: 5'-GTT GAA AAG CTG CTG GAA TCG NNN CCG GAC TGG CGT GAC
ACA G-3' (SEQ ID NO: 7)

Reverse primer: 5'-CTG TGT CAC GCC AGT CCG GNN NCG ATT CCA GCA GCT TTT
CAA C-3' (SEQ ID NO: 8)

The resulting extension product was digested with the DpnI restriction enzyme and transformed into DH5α competent cells to prepare *E. coli* strains comprising mutant genes. *E. coli* strains comprising the mutant genes were cultured, cells were then homogenized, and the supernatant of the homogenized cells was used to assay the SHNL activity and the protein concentration. SHNL activity was determined using DL-mandelonitrile as a substrate and determining the rate of benzaldehyde generation, which is generated by degrading mandelonitrile by an enzyme, based on changes in the absorbance at 249.6 nm. The protein concentration was determined using the BCA protein assay kit (Pierce) and BSA as a standard. Based on such results, the specific activity was determined, and the determined value was compared with the specific activity value obtained by culturing the *E*. *coli* Ver.0/pKK223-3/DH5α strains containing the Ver.0/pKK223-3 vector plasmid before mutagenesis. As a result of comparison, strains exhibiting improved specific activity were subjected to sequence analysis to identify mutated amino acids.

### [Results]

As a result of sequence analysis, SHNL in which amino acid 125 had been substituted with asparagine, cysteine, histidine, glutamine, arginine, leucine, isoleucine, threonine, or proline was found to exhibit significantly improved specific activity compared with the SHNL before mutagenesis (amino acid 125: phenylalanine) (Table 3).

**[Table 3]**

| Results of mutation of amino acid 125 | |
|---|---|
| Mutated amino acid | Specific activity* |
| Asn | 6.9 |
| Cys | 6.7 |
| His | 6.7 |
| Gln | 4.7 |
| Arg | 4.4 |
| Leu | 3.7 |
| Ile | 3.2 |
| Thr | 2.5 |
| Pro | 1.5 |

| | |
|---|---|
| *Specific activity = specific activity of mutant SHNL (U/mg-protein)/ specific activity of SHNL before mutagenesis (U/mg-protein) | |

### Example 3: Substitution of amino acid 216 of SHNL amino acid sequence

The results of Example 1 demonstrated that a high-activity mutant SHNL could be obtained by substituting glutamine 216 of the SHNL amino acid sequence with isoleucine. Accordingly, Site-directed mutagenesis was applied to the amino acid 216 of the SHNL amino acid sequence to substitute the amino acid 216 with any of various types of amino acids. Thus, further improvement in activity was attempted.

### [Material and method]

In the same manner as in Example 2, amino acid 216 of SHNL was substituted with any of various types of amino acids. Similarly, Ver.0 /pKK223-3 was used as a template. Primers shown below were used.

Forward primer: 5'-CAA AAT ATT CCT GCC GGA CTT CNN NCG CTG GCA AAT TGC
AAA CTA CA-3' (SEQ ID NO: 9)

Reverse primer: 5'-TGT AGT TTG CAA TTT GCC AGC GNN NGA AGT CCG GCA GGA
ATA TTT TG-3' (SEQ ID NO: 10)

The resulting DpnI-digested extension products were transformed into DH5α competent cells to prepare *E. coli* strains comprising mutant genes. The *E*. *coli* strains comprising the mutant genes were cultured, and specific activity thereof was compared with that of the *E*. *coli* Ver.0 /pKK223-3/DH5α strains comprising the genes before mutagenesis. As a result of comparison, strains exhibiting improved specific activity were subjected to sequence analysis to identify mutated amino acids.

### [Results]

All the clones selected as high-activity mutants were found to be SHNLs in which amino acid 216 had been substituted with histidine. Such clones exhibited significantly enhanced specific activity compared with glutamine (i.e., wild-type).

### Example 4: Substitution of amino acid 94 of SHNL amino acid sequence

The results of Example 1 demonstrate that a high-activity mutant SHNL could be obtained by substituting valine 94 of the SHNL amino acid sequence with alanine. Site-directed mutagenesis was applied to the amino acid 94 of the SHNL amino acid sequence to substitute the amino acid 94 with any of various types of amino acids. Thus, production of high-activity mutant SHNL was further attempted.

### [Material and method]

In the same manner as in Example 2, amino acid 94 of SHNL was substituted with any of various types of amino acids. Similarly, Ver.0 /pKK223-3 was used as a template. Primers shown below were used.

Forward primer: 5'-TGC TAT TGC TGC TGA TCG TTA CNN NGA CAA AAT TGC AGC
TGG CGT TT-3' (SEQ ID NO: 11)

Reverse primer: 5’- AAA CGC CAG CTG CAA TTT TGT CNN NGT AAC GAT CAG CAG
CAA TAG CA-3’ (SEQ ID NO: 12)

The resulting extension product was digested with the DpnI restriction enzyme and transformed into DH5α competent cells to prepare *E. coli* strains comprising mutant genes. *E. coli* strains comprising the mutant genes were cultured, the specific activity thereof was compared with that of the *E. coli* Ver.0/pKK223-3/DH5α strains comprising the genes before mutagenesis. As a result of comparison, strains exhibiting improved specific activity were subjected to sequence analysis to identify mutated amino acids.

### [Results]

As a result of sequence analysis, SHNL in which amino acid 94 had been substituted with alanine, cysteine, histidine, lysine, phenylalanine, proline, serine, threonine, tryptophan, or tyrosine was found to exhibit significantly improved specific activity compared with the SHNL before mutagenesis (amino acid 94: valine) (Table 4).

**[Table 4]**

| Results of mutation of amino acid 94 | |
|---|---|
| Mutated amino acid | Specific activity* |
| Ala | 11.9 |
| Cys | 6.4 |
| His | 8.4 |
| Lys | 7.9 |
| Phe | 4.6 |
| Pro | 14.3 |
| Ser | 7.4 |
| Thr | 11.8 |
| Trp | 6 |
| tyr | 5.3 |

| | |
|---|---|
| *Specific activity = specific activity of mutant SHNL (U/mg-protein)/ specific activity of SHNL before mutagenesis (U/mg-protein) | |

### Example 5: Substitution of amino acid 147 of SHNL amino acid sequence

The results of Example 1 demonstrate that a high-activity mutant SHNL could be obtained by substituting methionine 147 of the SHNL amino acid sequence with valine or isoleucine. Site-directed mutagenesis was applied to the amino acid 147 of the SHNL amino acid sequence to substitute the amino acid 147 with any of various types of amino acids. Thus, production of high-activity mutant SHNL was further attempted.

### [Material and method]

In the same manner as in Example 2, *E*. *coli* strains comprising mutant genes in which amino acid 147 of SHNL had been substituted with any of various types of amino acids were prepared. As a template, 10 ng of the SHNL-Wild/pET21a vector plasmid comprising the SHNL-Wild genes incorporated into the pET21 a vector (Novagen) was used, and extension reaction was carried out using the following primers.

Forward primer: 5'-GGC GAA ACC ATC ACT ACC NNN AAA CTG GGT TTC GTT CTG
CTG -3' (SEQ ID NO: 13)

Reverse primer: 5'-CAG CAG AAC GAA ACC CAG TTT NNN GGT AGT GAT GGT TTC
GCC-3' (SEQ ID NO: 14)

The vector plasmid after mutagenesis was digested with the DpnI restriction enzyme and transformed into BL21(DE3) cells. In the same manner as in Example 2, the specific activity thereof was compared with that of the *E*. *coli* SHNL-Wild/pET21a/BL21(DE3) strains comprising the genes before mutagenesis. As a result of comparison, strains exhibiting improved specific activity were subjected to sequence analysis to identify mutated amino acids.

### [Results]

As a result of sequence analysis, SHNL in which amino acid 147 had been substituted with threonine, serine, tyrosine, or cysteine was found to exhibit significantly enhanced specific activity compared with SHNL before mutagenesis (amino acid 147: methionine) (Table 5).

**[Table 5]**

| Results of mutation of amino acid 147 | |
|---|---|
| Mutated amino acid | Specific activity* |
| Thr | 2.6 |
| Ser | 2.4 |
| Tyr | 1.6 |
| Cys | 2.2 |

| | |
|---|---|
| *Specific activity = specific activity of mutant SHNL (U/mg-protein)/ specific activity of SHNL before mutagenesis (U/mg-protein) | |

### Comparative Example 1: Substitution of amino acid 128 of SHNL amino acid sequence

Mutant SHNLs with improved substrate receptivity of an enzyme (JP Patent Publication (kokai) No. 2000-125886 (A)) were prepared, and enzyme activity thereof was compared with that of Wild-SHNL.

### [Material and method]

In the same manner as in Example 2, *E*. *coli* strains comprising mutant genes in which tryptophan 128 of SHNL had been substituted with alanine were prepared. As a template, 10 ng of the SHNL-Wild/pET21a vector plasmid comprising the SHNL-Wild genes incorporated into the pET21a vector (Novagen) was used. For mutagenesis, primers having the sequences shown below were used. The vector plasmid after mutagenesis was digested with the DpnI restriction enzyme and then transformed into BL21(DE3). In the same manner as in Example 2, the specific activity of the resultant was compared with that of the *E. coli* SHNL-Wild/pET21a/BL21(DE3) strain comprising the gene before mutagenesis.

Forward primer: 5'-CTG CTG GAA TCG TTC CCG GAC GCA CGT GAC ACA GAA TAT
TTC ACG-3' (SEQ ID NO: 15)

Reverse primer: 5'-CGT GAA ATA TTC TGT GTC ACG TGC GTC CGG GAA CGA TTC
CAG CAG-3' (SEQ ID NO: 16)

### [Results]

The activity value of the Trp128Ala-SHNL enzyme with improved substrate receptivity of an enzyme was 25.5 U/ml-broth, and the specific activity value thereof was 8.9 U/mg-protein. The specific activity value of the Wild-SHNL enzyme that had been cultured under the same conditions was 9.8 U/mg-protein. Specifically, under the assay conditions employed in the experiment, the activity value of the Trp128Ala-SHNL enzyme was found to be equivalent to that of the Wild-SHNL enzyme. Also, improvement in the activity value of the mutants obtained in Example 1 in which tryptophan 128 had been substituted with leucine, cysteine, or serine (i.e., 005F6, 033D10, and 046D9 strains shown in Table 2) was about 1.1 to 2 times that of the Wild-SHNL enzyme. According to the present invention, mutant enzymes with activity 2 to 10 times higher than that a wild-type strain were obtained, as demonstrated in Examples 1 to 5. Accordingly, the high-activity mutant SHNL of the present invention was demonstrated to be very useful.

### Example 6: Substitution of amino acid 122 of SHNL amino acid sequence

The results of Examples 1 to 5 demonstrate that a high-activity mutant SHNL could be obtained by substituting amino acid at a given site of the SHNL with another amino acid. Based on the conformational data of SHNL registered at the Protein Data Bank (PDB), Site-directed mutagenesis was applied to amino acid 122 located in the vicinity of the mutation site of the high-activity mutant SHNL that had been already obtained to substitute an amino acid of interest with any of various types of amino acids. Thus, production of high-activity mutant SHNL was further attempted.

### [Material and method]

In the same manner as in Example 2, recombinant *E. coli* strains comprising mutant genes in which amino acid 122 of SHNL had been substituted with any of various types of amino acids were prepared. As a template, 10 ng of the Ver.0/pET21a plasmid comprising the SHNL-Wild genes incorporated into the pET21a vector (Novagen) was used. Primers shown below were used.

Forward primer: 5'-GTC TTA CAC TGT TGA AAA GCT GNN NGA ATC GTT CCC GGA
CTG GCG TG-3' (SEQ ID NO: 17)

Reverse primer: 5'- CAC GCC AGT CCG GGA ACG ATT CNN NCA GCT TTT CAA CAG
TGT AAG AC-3' (SEQ ID NO: 18)

The plasmid after mutagenesis was transformed into BL21(DE3). In the same manner as in Example 2, the specific activity of the resultant was compared with that of the SHNL-Wild/pET21a/BL21(DE3) strain before mutagenesis. The strains exhibiting improved specific activity were subjected to sequence analysis to identify the mutated amino acid.

### [Results]

SHNL in which amino acid 122 had been substituted with isoleucine, valine, glycine, or serine exhibited improved specific activity compared with wild-type SHNL (amino acid 122: leucine) before mutagenesis (Table 6).

**[Table 6]**

| Results of mutation of amino acid 122 | |
|---|---|
| Mutated amino acid | Specific activity* |
| Ile | 2.1 |
| Ser | 2.4 |
| Val | 1.5 |
| Gly | 1.6 |

| | |
|---|---|
| *Specific activity = specific activity of mutant SHNL (U/mg-protein)/ specific activity of SHNL before mutagenesis (U/mg-protein) | |

### Example 7: Insertion of amino acid between amino acids 128 and 129 of SHNL amino acid sequence

### [Material and method]

In the same manner as in Example 2, *E*. *coli* strains comprising mutant genes in which a new amino acid had been inserted between amino acids 128 and 129 of SHNL were prepared. As a template, 10 ng of the SHNL-Wild/ pET21a vector plasmid comprising the SHNL-Wild genes incorporated into the pET21a vector was used. Primers shown below were used.

Forward primer: 5'-GAA TCG TTC CCG GAC TGG NNN CGT GAC ACA GAA TAT
TTC-3' (SEQ ID NO: 19)

Reverse primer: 5'-GAA ATA TTC TGT GTC ACG NNN CCA GTC CGG GAA CGA
TTC-3' (SEQ ID NO: 20)

The vector plasmid after mutagenesis was digested with the DpnI restriction enzyme and then transformed into BL21(DE3). In the same manner as in Example 2, the specific activity of the resultant was compared with that of the *E*. *coli* SHNL-Wild/pET21a/BL21(DE3) strain comprising the gene before mutagenesis. The strains exhibiting improved specific activity were subjected to sequence analysis to identify the inserted amino acid.

### [Results]

As a result of sequence analysis, SHNL in which glycine or lysine had been inserted between amino acid 128 and 129 exhibited improved specific activity compared with wild-type SHNL before mutagenesis (Table 7).

**[Table 7]**

| Results of amino acid insertion between amino acids 128 and 129 | |
|---|---|
| Inserted amino acid | Specific activity* |
| Lys | 1.6 |
| Gly | 1.4 |

| | |
|---|---|
| *Specific activity = specific activity of mutant SHNL (U/mg-protein)/ specific activity of SHNL before mutagenesis (U/mg-protein) | |

### Example 8: Construction of high-activity mutant SHNL via combination of mutation sites and culture using jar fermenter

### [Material and method]

In the same manner as in Example 2, site-directed mutagenesis was applied to the Ver.0 gene using the QuikChange XL Site-Directed Mutagenesis Kit to obtain a high-activity mutant SHNL gene comprising combined mutation sites encoding SHNL in which amino acids 94, 122, 125, and 216 of SHNL had been substituted with any of various types of amino acids. A pET21a vector plasmid comprising the high-activity mutant SHNL gene comprising combined mutation sites obtained by this method was transformed into BL21(DE3) or BL21(DE3)Ster (Novagen) competent cells to prepare *E. coli* strains comprising high-activity mutant SHNL gene comprising combined mutation sites.

The resulting *E*. *coli* strains comprising the high-activity mutant SHNL gene comprising combined mutation sites were inoculated in 5 ml of LB medium, and reactivation culture was carried out at 37°C for 16 hours. The reactivation culture medium (2 ml) was inoculated on 50 ml of NS-2 medium shown in Table 8 to perform seed culture at 15°C for 48 hours.

**[Table 8]**

| Composition of NS-2 medium | |
|---|---|
| Glycerol | 40 g |
| (NH₄)₂SO₄ | 10 g |
| KH₂PO₄ | 2g |
| K₂HPO₄ | 6g |
| Yeast ext. (Ebios P2G) | 40 g |
| MgSO₄·7H₂O | 1 g |
| Ampicillin | 1 g |
| IPTG | 0.238 g |
| Distilled water | 1 liter |

The obtained seed culture medium (24 ml) was inoculated in 1.2 1 of NS-2 medium, and culture was carried out using a 2-1-jar fermenter. Culture was carried out at 15°C, an aeration rate of 1 vvm (1.2 l/min), and an agitation rate of 680 rpm. Culture was terminated when the stationary phase was attained, and the obtained culture medium was centrifuged at 6,000x G for 30 minutes to recover cells. The recovered cells were weighed, 0.2 M Na-citrate buffer (pH 5.5) was added thereto in an amount twice by weight the cells to allow the cells to suspend therein, and cells were subjected to homogenization three times using a pressurized homogenizer at 700 to 800 bar. The solution of homogenized cells were heated at 50°C for 2 hours, and centrifuged at 6,000x G for 30 minutes to obtain a supernatant of homogenized cells. The SHNL activity and the protein concentration were assayed using the supernatant of homogenized cells. Based on the results, the specific activity was determined, and the determined value was compared with the specific activity attained by culturing the *E. coli* Ver.0 /pET21a/BL21(DE3) strains comprising the genes before mutagenesis. As a result of comparison, strains exhibiting improved specific activity were subjected to sequence analysis to identify the substituted amino acid. For comparison, SHNL-W128A/pET21a/BL21(DE3) constructed in Comparative Example 1 was cultured in the same manner, and the specific activity was determined.

### [Results]

The *E. coli* strains comprising the high-activity mutant SHNL gene comprising combined mutation sites exhibited higher specific activity than *E*. *coli* strains comprising a vector plasmid before mutagenesis and *E*. *coli* strains comprising the high-activity SHNL genes comprising a single amino acid substitution. The *E*. *coli* strains exhibiting the highest specific activity were: the *E. coli* Ver.1+L122S+Q216H-2/pET21a/BL21(DE3) strains comprising a mutant SHNL plasmid vector obtained by combining an additional 3 high-activity mutations (i.e., substitution of phenylalanine 125 with asparagine, that of leucine 122 with serine, and that of glutamine 216 with histidine) to Ver.0/pET21a; and the *E*. *coli* Ver.1+L122S-2/pET21a/BL21(DE3) strains comprising a mutant SHNL plasmid vector obtained by combining an additional 2 high-activity mutations (i.e., substitution of phenylalanine 125 with asparagine and that of leucine 122 with serine) to Ver.0/pET21a (Table 9). The specific activity levels thereof exceeded 50 U/mg-protein, which was at least 5 times higher than that of the *E*. *coli* SHNL-Gly165E,V173L/pET21a/BL21(DE3) strains comprising a vector plasmid before mutagenesis. However, the results attained from the SHNL-W128A/pET21a/BL21(DE3) constructed in Comparative Example 1 were lower than the results attained from the E. coli strain comprising a vector plasmid before mutagenesis.

**[Table 9]**

| Comparison of activity of mutant enzymes cultured in jar fermenter | | | | | | | | | | | |
|---|---|---|---|---|---|---|---|---|---|---|---|
| Strains | Genotype | | | | | | Duration of culture | Cell density | Activity | Protein | Specific activity |
| | | | | | | | (h) | 660nm | u/ml | mg/ml | u/mg |
| Ver.0/pET21a/BL21(DE3) | G165E | V173L | | | | | 52 | 58.24 | 211.7 | 22.8 | 9.3 |
| Ver.0+F125N-1/pET21a/BL21(DE3)Star | G165E | V173L | F125N | | | | 60 | 43.3 | 802.7 | 20.3 | 39.6 |
| Ver.0+F125H-2/pET21a/BL21(DE3)Star | G165E | V173L | F125H | | | | 72 | 42.8 | 836.6 | 21.1 | 39.7 |
| Ver.0+F125L-1/pET21a/BL21(DE3)Star | G165E | V173L | F125L | | | | 78 | 41.43 | 772.0 | 20.2 | 38.2 |
| Ver.0+F125R-1/pET21a/BL21(DE3)Star | G165E | V173L | F125R | | | | 78 | 44.31 | 823.0 | 21.4 | 38.5 |
| Ver.0+F125Q-1 /pET21 a/BL21 (DE3)Star | G165E | V173L | F125Q | | | | 78 | 25.5 | 635.0 | 22.0 | 28.8 |
| Ver.0+F125C-1 /pET21 a/BL21 (DE3)Star | G165E | V173L | F125C | | | | 78 | 32.46 | 788.0 | 20.6 | 38.3 |
| Ver.0+F125N-1/pET21a/BL21(DE3) | G165E | V173L | F125N | | | | 54.5 | 45.66 | 640.5 | 24.2 | 26.5 |
| Ver.1+L1221-2/pET21a/BL21(DE3) | G165E | V173L | F125N | L1221 | | | 60 | 39.6 | 594.0 | 22.4 | 26.5 |
| Ver.1+L122S-2/pET21a/BL21(DE3) | G165E | V173L | F125N | L122S | | | 60 | 41.94 | 1148.0 | 21.6 | 53.1 |
| Ver.1+L1221+Q216H-1/pET21a/BL21(DE3) | G165E | V173L | F125N | L122I | Q216H | | 80 | 40.08 | 789.0 | 19.8 | 39.9 |
| Ver.1+L122S+Q216H-2/pET21a/BL21(DE3) | G165E | V173L | F125N | L122S | Q216H | | 54 | 41.28 | 1053.0 | 19.6 | 53.8 |
| Ver.3+V94A-1/pET21a/BL21(DE3) | G165E | V173L | F125N | L122S | Q216H | V94A | 60 | 40.8 | 875.0 | 23.0 | 38.0 |
| Ver.3+V94P-4/pET21a/BL21(DE3) | G165E | V173L | F125N | L122S | Q216H | V94P | 70 | 38.34 | 643.0 | 18.0 | 35.7 |
| Ver.3+V94T-1/pET21a/BL21(DE3) | G165E | V173L | F125N | L122S | Q216H | V94T | 67 | 38.52 | 806.0 | 24.7 | 32.6 |
| Ver.3+V94S-1/pET21a/BL21(DE3) | G165E | V173L | F125N | L122S | | Q216H V94S | 48 | 49.8 | 1064.0 | 22.6 | 47.1 |
| Ver.O+L1221-1/pET21a/BL21(DE3)Star | G165E | V173L | | L1221 | | | 72 | 51.84 | 645.8 | 36.1 | 17.9 |
| Ver.0+L122S-1/pET21a/BL21(DE3)Star | G165E | V173L | | L122S | | | 72 | 33.54 | 569.1 | 26.7 | 21.3 |
| Ver.0+L122G-2/pET21a/BL21(DE3)Star | G165E | V173L | | L122G | | | 70 | 35.58 | 269.0 | 18.7 | 14.4 |
| Ver.0+L122V-1/pET21a/BL21(DE3)Star | G165E | V173L | | L122V | | | 76.45 | 41.52 | 454.6 | 20.7 | 22.0 |
| W128A/pET21a/BL21(DE3) | | | | | W128A | | 61 | 33.66 | 123.8 | 24.8 | 5.0 |

### Example 9: Production of optically active cyanohydrin using high-activity mutant comprising combined mutations

Whether or not the high-activity mutant SHNL comprising combined mutation sites could produce optically active cyanohydrin as with common SHNL was inspected.

### [Material and method]

### 1) Preparation of immobilized enzyme

0.2 M Na-citrate buffer (pH 5.5) was added to the supernatant of homogenized Ver.1+L122I-2/pET21a/BL21(DE3) cells obtained in Example 8, and the activity level was adjusted at 500 U/ml. Silica gel (300 mg) was mixed with 0.3 ml of the enzyme solution to obtain immobilized enzymes. The supernatant of homogenized *E*. *coli* Ver.0/pET21a/BL21(DE3) cells comprising the SHNL gene before mutagenesis was subjected to the same procedure to prepare immobilized enzymes.

### 2) Enzyme reaction

To t-butylmethylether (4.492 ml) comprising 1.5 M HCN dissolved therein, 0.337 ml of a 0.2 M citrate buffer (pH 5.5) was added, the resulting mixture was agitated for 30 minutes and allowed to stand thereafter, and the separated aqueous phase was removed. The solution was added to a 9-ml screw vial containing 300 mg of the immobilized enzymes prepared above. 2-Chlorobenzaldehyde was added to a final concentration of 1.0 M therein, and an enzyme reaction was carried out by stirring. After the completion of the reaction, the reaction solution was recovered, and the substrate conversion rate and the optical purity were assayed.

### [Results]

The supernatant of the homogenized Ver.1+L122I-2/pET21aBL21(DE3) cells exhibited the conversion rate and the optical purity substantially the same as those of the strain before mutagenesis (Table 10). Thus, the Ver.1+L122I-2 SHNL strains, i.e., the high-activity enzymes comprising combined mutations, were found to be capable of producing optically active cyanohydrin as with the mutant SHNL that the present inventors had produced in the past.

**[Table 10]**

| Synthesis of S-2-chloromandelonitrile using mutant enzyme | | | |
|---|---|---|---|
| Enzyme strain used | Genotype | Conversion rate (mol%) | Optical purity (%ee) |
| Ver.0/pET21a/BL2(DE3) | G165E V173L | 97.2 | 95.5 |
| Ver.1+L122I-2/pET21a/BL21(DE3) | G165E V173L F125N L122I | 91.5 | 95.8 |

### Reference Example: Preparation of Ver.0/pKK223-3

### 1) Substitution of Val 173 with Leu

SHNL in which amino acid 173 had been substituted with Leu was prepared using the QuikChange XL Site-Directed Mutagenesis kit (Stratagene).

As a template, 10 ng of the SHNL-Wild/pKK223-3 vector plasmid was used, and extension reaction was carried out using the following oligo DNAs as the primers.

Forward primer: 5'-GGC GAA TAT GAA CTG GCA AAA ATG CTG ATG CGC AAG GGC
TCT CTG-3' (SEQ ID NO: 21)

Reverse primer: 5'-CAG AGA GCC CTT GCG CAT CAG CAT TTT TGC CAG TTC ATA
TTC GCC-3' (SEQ ID NO: 22)

Subsequently, the obtained reaction product was digested with the DpnI restriction enzyme included in the kit, the resulting restriction enzyme-digested reaction product was transformed into DH5α competent cells to prepare the SHNL-V173L/pKK223-3/DH5α strain.

### 2) Substitution of glycine 165 with glutamine

The SHNL-V173L/pKK223-3 plasmid was prepared from the resulting SHNL-V173L/pKK223-3/DH5α strain, and site-directed mutagenesis was performed again using the resulting strain as a template. Extension reaction was carried out using the following primers, and the resulting reaction product was digested with the DpnI restriction enzyme.

Forward primer: 5’-CGT GAA AAC CTG TTC ACC AAA TGC ACT GAT GAA GAA TAT
GAA CTG GCA AAA ATG-3’ (SEQ ID NO: 23)

Reverse primer: 5'-CAT TTT TGC CAG TTC ATA TTC TTC ATC AGT GCA TTT GGT
GAA CAG GTT TTC ACG-3' (SEQ ID NO: 24)

The resulting restriction enzyme-digested reaction product was transformed into DH5α competent cells to prepare the SHNL-V173L/pKK223-3/DH5α strain. The plasmid was prepared from the culture medium, and the Ver.0/pKK223-3 plasmid comprising the SHNL gene having 2 amino acid mutations, Gly165Glu and Val173Leu, was obtained.

All publications, patents, and patent applications cited herein are incorporated herein by reference in their entirety.

### Industrial Applicability

The high-activity modified SHNL of the present invention has higher enzyme activity than wild-type SHNL, and it can effectively synthesize optically active cyanohydrin with high optical purity. Accordingly, the high-activity modified SHNL of the present invention is very useful as an enzyme for producing optically active cyanohydrin at industry levels.

### Sequence Listing Free Text

SEQ ID NO: 5; description of an artificial sequence: primer
SEQ ID NO: 6; description of an artificial sequence: primer
SEQ ID NO: 7; description of an artificial sequence: primer
SEQ ID NO: 8; description of an artificial sequence: primer
SEQ ID NO: 9; description of an artificial sequence: primer
SEQ ID NO: 10; description of an artificial sequence: primer
SEQ ID NO: 11; description of an artificial sequence: primer
SEQ ID NO: 12; description of an artificial sequence: primer
SEQ ID NO: 13; description of an artificial sequence: primer
SEQ ID NO: 14; description of an artificial sequence: primer
SEQ ID NO: 15; description of an artificial sequence: primer
SEQ ID NO: 16; description of an artificial sequence: primer
SEQ ID NO: 17; description of an artificial sequence: primer
SEQ ID NO: 18; description of an artificial sequence: primer
SEQ ID NO: 19; description of an artificial sequence: primer
SEQ ID NO: 20; description of an artificial sequence: primer
SEQ ID NO: 21; description of an artificial sequence: primer
SEQ ID NO: 22; description of an artificial sequence: primer
SEQ ID NO: 23; description of an artificial sequence: primer
SEQ ID NO: 24; description of an artificial sequence: primer
SEQ ID NO: 25; DNA encoding mutant SHNL in which glycine 165 and valine 173 had been substituted with glutamine and leucine, respectively

## Claims

1. Modified S-hydroxynitrile lyase, which is obtained by substituting at least one amino acid selected from amino acids 14, 44, 66, 94, 103, 118, 122, 125, 127, 129, 147, 148, 152, 212, and 216 in the amino acid sequence (SEQ ID NO: 2) of wild-type S-hydroxynitrile lyase derived from cassava (*Manihot esculenta*) with another amino acid, or modified S-hydroxynitrile lyase, which is obtained by substituting at least one amino acid selected from amino acids 14, 44, 66, 94, 103, 118, 122, 125, 127, 129, 146, 147, 151, 211, and 215 in the amino acid sequence (SEQ ID NO: 4) of wild-type S-hydroxynitrile lyase derived from the Pará rubber tree (*Hevea brasiliensis*) with another amino acid.

2. Modified S-hydroxynitrile lyase, which is obtained by inserting at least one amino acid between amino acids 128 and 129 of the amino acid sequence (SEQ ID NO: 2 or 4) of the cassava (*Manihot esculenta*)- or Pará rubber tree (*Hevea brasiliensis*)*-*derived wild-type S-hydroxynitrile lyase.

3. Modified S-hydroxynitrile lyase comprising at least one amino acid substitution selected from among the substitutions shown below in the amino acid sequence (SEQ ID NO: 2) of wild-type S-hydroxynitrile lyase derived from cassava (*Manihot esculenta*):
a) substitution of histidine 14 with glutamine;
b) substitution of aspartic acid 44 with asparagine;
c) substitution of glutamic acid 66 with aspartic acid or lysine;
d) substitution of valine 94 with alanine, cysteine, histidine, lysine, phenylalanine, proline, serine, threonine, tryptophan, or tyrosine;
e) substitution of histidine 103 with glutamine;
f) substitution of valine 118 with leucine;
g) substitution of leucine 122 with isoleucine, valine, glycine, or serine;
h) substitution of phenylalanine 125 with asparagine, cysteine, histidine, glutamine, arginine, leucine, isoleucine, threonine, or proline;
i) substitution of aspartic acid 127 with glutamic acid, asparagine, or glycine;
j) substitution of arginine 129 with histidine;
k) substitution of methionine 147 with threonine, serine, tyrosine, or cysteine;
l) substitution of lysine 148 with asparagine;
m) substitution of valine 152 with alanine or glycine;
n) substitution of leucine 212 with proline; and
o) substitution of glutamine 216 with histidine.

4. Modified S-hydroxynitrile lyase, which is obtained by inserting glycine or lysine between amino acids 128 and 129 of the amino acid sequence (SEQ ID NO: 2 or 4) of the cassava (*Manihot esculenta*)- or Pará rubber tree (*Hevea brasiliensis*)-derived wild-type S-hydroxynitrile lyase.

5. The modified S-hydroxynitrile lyase according to claim 3 or 4, which further comprises substitution of glycine 165 with glutamic acid and substitution of valine 173 with leucine.

6. DNA encoding the amino acid sequence of the modified S-hydroxynitrile lyase according to any one of claims 1 to 5.

7. A method for producing modified S-hydroxynitrile lyase comprising culturing a host into which the DNA according to claim 6 has been introduced and recovering a protein having S-hydroxynitrile lyase activity from the resulting culture product.

8. A method for producing optically active cyanohydrin comprising allowing the modified S-hydroxynitrile lyase according to any one of claims 1 to 5 to react with a carbonyl compound and cyanide.


SEQUENCE LISTING

<110>  NIPPON SHOKUBAI CO., LTD.

<120>  Novel high-activity modified S-hydroxynitrile lyase

<130>  P033591EP

<140>  06810829.9
<141>  2006-09-29

<160>  26

<170>  PatentIn version 3.5

<210>  1
<211>  777
<212>  DNA
<213>  Manihot esculenta


<220>
<221>  CDS
<222>  (1)..(777)

<400>  1
atg gtt act gca cac ttc gtt ctg att cac acc att tgt cac ggc gca      48
Met Val Thr Ala His Phe Val Leu Ile His Thr Ile Cys His Gly Ala
1               5                   10                  15

tgg att tgg cac aaa ctg aaa ccg gcc ctg gaa cgt gct ggc cac aaa      96
Trp Ile Trp His Lys Leu Lys Pro Ala Leu Glu Arg Ala Gly His Lys
            20                  25                  30

gtt act gca ctg gac atg gca gcc agt ggc att gac ccg cgt caa att      144
Val Thr Ala Leu Asp Met Ala Ala Ser Gly Ile Asp Pro Arg Gln Ile
        35                  40                  45

gaa cag atc aac tct ttc gat gaa tac tct gaa ccg ctg ctg act ttc      192
Glu Gln Ile Asn Ser Phe Asp Glu Tyr Ser Glu Pro Leu Leu Thr Phe
    50                  55                  60

ctg gaa aaa ctg ccg caa ggc gaa aag gtt atc att gtt ggt gaa agc      240
Leu Glu Lys Leu Pro Gln Gly Glu Lys Val Ile Ile Val Gly Glu Ser
65                  70                  75                  80

tgt gca ggc ctg aac att gct att gct gct gat cgt tac gtt gac aaa      288
Cys Ala Gly Leu Asn Ile Ala Ile Ala Ala Asp Arg Tyr Val Asp Lys
                85                  90                  95

att gca gct ggc gtt ttc cac aac tcc ctg ctg ccg gac acc gtt cac      336
Ile Ala Ala Gly Val Phe His Asn Ser Leu Leu Pro Asp Thr Val His
            100                 105                 110

agc ccg tct tac act gtt gaa aag ctg ctg gaa tcg ttc ccg gac tgg      384
Ser Pro Ser Tyr Thr Val Glu Lys Leu Leu Glu Ser Phe Pro Asp Trp
        115                 120                 125

cgt gac aca gaa tat ttc acg ttc acc aac atc act ggc gaa acc atc      432
Arg Asp Thr Glu Tyr Phe Thr Phe Thr Asn Ile Thr Gly Glu Thr Ile
    130                 135                 140

act acc atg aaa ctg ggt ttc gtt ctg ctg cgt gaa aac ctg ttc acc      480
Thr Thr Met Lys Leu Gly Phe Val Leu Leu Arg Glu Asn Leu Phe Thr
145                 150                 155                 160

aaa tgc act gat ggc gaa tat gaa ctg gca aaa atg gtt atg cgc aag      528
Lys Cys Thr Asp Gly Glu Tyr Glu Leu Ala Lys Met Val Met Arg Lys

                165                 170                 175

ggc tct ctg ttc caa aac gtt ctg gct cag cgt ccg aag ttc act gaa 576
Gly Ser Leu Phe Gln Asn Val Leu Ala Gln Arg Pro Lys Phe Thr Glu
            180                 185                 190

aaa ggc tac ggc tct atc aag aaa gtt tat att tgg acc gat caa gac 624
Lys Gly Tyr Gly Ser Ile Lys Lys Val Tyr Ile Trp Thr Asp Gln Asp
        195                 200                 205

aaa ata ttc ctg ccg gac ttc caa cgc tgg caa att gca aac tac aaa 672
Lys Ile Phe Leu Pro Asp Phe Gln Arg Trp Gln Ile Ala Asn Tyr Lys
    210                 215                 220

ccg gac aag gtt tat cag gtt caa ggc ggc gat cac aag ctg cag ctg 720
Pro Asp Lys Val Tyr Gln Val Gln Gly Gly Asp His Lys Leu Gln Leu
225                 230                 235                 240

aca aaa act gaa gaa gta gct cac att ctg caa gaa gtt gct gat gca 768
Thr Lys Thr Glu Glu Val Ala His Ile Leu Gln Glu Val Ala Asp Ala
                245                 250                 255

tac gct taa 777
Tyr Ala

<210> 2
<211> 258
<212> PRT
<213> Manihot esculenta

<400> 2

Met Val Thr Ala His Phe Val Leu Ile His Thr Ile Cys His Gly Ala
1               5                   10                  15

Trp Ile Trp His Lys Leu Lys Pro Ala Leu Glu Arg Ala Gly His Lys
            20                  25                  30

Val Thr Ala Leu Asp Met Ala Ala Ser Gly Ile Asp Pro Arg Gln Ile
        35                  40                  45

Glu Gln Ile Asn Ser Phe Asp Glu Tyr Ser Glu Pro Leu Leu Thr Phe
    50                  55                  60

Leu Glu Lys Leu Pro Gln Gly Glu Lys Val Ile Ile Val Gly Glu Ser
65                  70                  75                  80

Cys Ala Gly Leu Asn Ile Ala Ile Ala Ala Asp Arg Tyr Val Asp Lys
                85                  90                  95

Ile Ala Ala Gly Val Phe His Asn Ser Leu Leu Pro Asp Thr Val His
            100                 105                 110

Ser Pro Ser Tyr Thr Val Glu Lys Leu Leu Glu Ser Phe Pro Asp Trp
        115                 120                 125

Arg Asp Thr Glu Tyr Phe Thr Phe Thr Asn Ile Thr Gly Glu Thr Ile
    130                 135                 140

Thr Thr Met Lys Leu Gly Phe Val Leu Leu Arg Glu Asn Leu Phe Thr
145                 150                 155                 160

Lys Cys Thr Asp Gly Glu Tyr Glu Leu Ala Lys Met Val Met Arg Lys
                165                 170                 175

Gly Ser Leu Phe Gln Asn Val Leu Ala Gln Arg Pro Lys Phe Thr Glu
            180                 185                 190

Lys Gly Tyr Gly Ser Ile Lys Lys Val Tyr Ile Trp Thr Asp Gln Asp
        195                 200                 205

Lys Ile Phe Leu Pro Asp Phe Gln Arg Trp Gln Ile Ala Asn Tyr Lys
    210                 215                 220

Pro Asp Lys Val Tyr Gln Val Gln Gly Gly Asp His Lys Leu Gln Leu
225                 230                 235                 240

Thr Lys Thr Glu Glu Val Ala His Ile Leu Gln Glu Val Ala Asp Ala
                245                 250                 255

Tyr Ala

<210>  3
<211>  774
<212>  DNA
<213>  Hevea brasiliensis

<220>
<221>  CDS
<222>  (1)..(774)

<400>  3
atg gca ttc gct cat ttt gtt ctt att cat acc ata tgc cac ggt gca      48
Met Ala Phe Ala His Phe Val Leu Ile His Thr Ile Cys His Gly Ala
1               5                   10                  15

tgg att tgg cac aag ctc aaa ccc ctc ctt gag gca ctt ggc cac aag      96
Trp Ile Trp His Lys Leu Lys Pro Leu Leu Glu Ala Leu Gly His Lys
            20                  25                  30

gtt act gca ctg gac ctt gca gca agc ggc gtt gac cca agg caa att      144
Val Thr Ala Leu Asp Leu Ala Ala Ser Gly Val Asp Pro Arg Gln Ile
        35                  40                  45

gag gag att ggc tca ttt gat gag tat tct gaa ccc ttg ttg acg ttc      192
Glu Glu Ile Gly Ser Phe Asp Glu Tyr Ser Glu Pro Leu Leu Thr Phe
    50                  55                  60

ttg gag gca ctc cct cca ggg gaa aag gtg att ctg gtt ggc gag agc      240
Leu Glu Ala Leu Pro Pro Gly Glu Lys Val Ile Leu Val Gly Glu Ser
65                  70                  75                  80

tgt gga gga ctc aat ata gca att gct gct gat aaa tac tgt gaa aag      288
Cys Gly Gly Leu Asn Ile Ala Ile Ala Ala Asp Lys Tyr Cys Glu Lys
                85                  90                  95

att gca gct gct gtt ttc cac aat tca gta ttg cca gac acc gag cac 336
Ile Ala Ala Ala Val Phe His Asn Ser Val Leu Pro Asp Thr Glu His
            100                 105                 110

tgc cca tct tac gtc gtg gat aag ctc atg gag gtg ttt ccc gac tgg 384
Cys Pro Ser Tyr Val Val Asp Lys Leu Met Glu Val Phe Pro Asp Trp
        115                 120                 125

aaa gac acc acg tat ttt acg tac act aaa gat ggc aag gag ata act 432
Lys Asp Thr Thr Tyr Phe Thr Tyr Thr Lys Asp Gly Lys Glu Ile Thr
    130                 135                 140

gga ttg aaa ctg ggc ttc acg ctt ctg agg gaa aat tta tat acc ctt 480
Gly Leu Lys Leu Gly Phe Thr Leu Leu Arg Glu Asn Leu Tyr Thr Leu
145                 150                 155                 160

tgc ggt cct gag gaa tat gaa ctg gcg aag atg ttg aca agg aag gga 528
Cys Gly Pro Glu Glu Tyr Glu Leu Ala Lys Met Leu Thr Arg Lys Gly
                165                 170                 175

tca tta ttt caa aat att tta gct aag cga cca ttc ttc act aag gaa 576
Ser Leu Phe Gln Asn Ile Leu Ala Lys Arg Pro Phe Phe Thr Lys Glu
            180                 185                 190

ggt tac gga tcg att aag aaa att tat gtg tgg acc gac caa gac gaa 624
Gly Tyr Gly Ser Ile Lys Lys Ile Tyr Val Trp Thr Asp Gln Asp Glu
        195                 200                 205

ata ttt tta cct gaa ttt caa ctc tgg caa ata gaa aac tat aaa cca 672
Ile Phe Leu Pro Glu Phe Gln Leu Trp Gln Ile Glu Asn Tyr Lys Pro
    210                 215                 220

gac aag gtt tat aag gtc gaa ggt gga gat cat aaa ttg cag ctt aca 720
Asp Lys Val Tyr Lys Val Glu Gly Gly Asp His Lys Leu Gln Leu Thr
225                 230                 235                 240

aag act aag gag atc gct gaa att ctc caa gag gtg gct gat acc tat 768
Lys Thr Lys Glu Ile Ala Glu Ile Leu Gln Glu Val Ala Asp Thr Tyr
                245                 250                 255

aat tga 774
Asn

<210> 4
<211> 257
<212> PRT
<213> Hevea brasiliensis

<400> 4

Met Ala Phe Ala His Phe Val Leu Ile His Thr Ile Cys His Gly Ala
1               5                   10                  15

Trp Ile Trp His Lys Leu Lys Pro Leu Leu Glu Ala Leu Gly His Lys
            20                  25                  30

Val Thr Ala Leu Asp Leu Ala Ala Ser Gly Val Asp Pro Arg Gln Ile
        35                  40                  45

Glu Glu Ile Gly Ser Phe Asp Glu Tyr Ser Glu Pro Leu Leu Thr Phe
    50                  55                  60

Leu Glu Ala Leu Pro Pro Gly Glu Lys Val Ile Leu Val Gly Glu Ser
65                  70                  75                  80

Cys Gly Gly Leu Asn Ile Ala Ile Ala Ala Asp Lys Tyr Cys Glu Lys
                85                  90                  95

Ile Ala Ala Ala Val Phe His Asn Ser Val Leu Pro Asp Thr Glu His
            100                 105                 110

Cys Pro Ser Tyr Val Val Asp Lys Leu Met Glu Val Phe Pro Asp Trp
        115                 120                 125

Lys Asp Thr Thr Tyr Phe Thr Tyr Thr Lys Asp Gly Lys Glu Ile Thr
    130                 135                 140

Gly Leu Lys Leu Gly Phe Thr Leu Leu Arg Glu Asn Leu Tyr Thr Leu
145                 150                 155                 160

Cys Gly Pro Glu Glu Tyr Glu Leu Ala Lys Met Leu Thr Arg Lys Gly
                165                 170                 175

Ser Leu Phe Gln Asn Ile Leu Ala Lys Arg Pro Phe Phe Thr Lys Glu
            180                 185                 190

Gly Tyr Gly Ser Ile Lys Lys Ile Tyr Val Trp Thr Asp Gln Asp Glu
        195                 200                 205

Ile Phe Leu Pro Glu Phe Gln Leu Trp Gln Ile Glu Asn Tyr Lys Pro
    210                 215                 220

Asp Lys Val Tyr Lys Val Glu Gly Gly Asp His Lys Leu Gln Leu Thr
225                 230                 235                 240

Lys Thr Lys Glu Ile Ala Glu Ile Leu Gln Glu Val Ala Asp Thr Tyr
                245                 250                 255

Asn

<210>  5
<211>  42
<212>  DNA
<213>  Artificial Sequence

<220>
<223>  Primer

<400>  5
gggggggaat tcatggttac tgcacacttc gttctgattc ac                          42

<210>  6
<211>  42
<212>  DNA

<213>  Artificial Sequence

<220>
<223>  Primer

<400>  6
ggggggaagc ttttaagcgt atgcatcagc aacttcttgc ag                                42

<210>  7
<211>  43
<212>  DNA
<213>  Artificial Sequence

<220>
<223>  Primer

<220>
<221>  misc_feature
<222>  (22)..(24)
<223>  n is a, c, g, or t

<400>  7
gttgaaaagc tgctggaatc gnnnccggac tggcgtgaca cag                              43

<210>  8
<211>  43
<212>  DNA
<213>  Artificial Sequence

<220>
<223>  Primer

<220>
<221>  misc_feature
<222>  (20)..(22)
<223>  n is a, c, g, or t

<400>  8
ctgtgtcacg ccagtccggn nncgattcca gcagcttttc aac                              43

<210>  9
<211>  47
<212>  DNA
<213>  Artificial Sequence

<220>
<223>  Primer

<220>
<221>  misc_feature
<222>  (23)..(25)
<223>  n is a, c, g, or t

<400>  9
caaaatattc ctgccggact tcnnncgctg gcaaattgca aactaca                          47

<210>  10
<211>  47
<212>  DNA
<213>  Artificial Sequence

<220>
<223>  Primer


<220>
<221>  misc_feature
<222>  (23)..(25)
<223>  n is a, c, g, or t

<400>  10
tgtagtttgc aatttgccag cgnnngaagt ccggcaggaa tattttg                    47


<210>  11
<211>  47
<212>  DNA
<213>  Artificial Sequence

<220>
<223>  Primer


<220>
<221>  misc_feature
<222>  (23)..(25)
<223>  n is a, c, g, or t

<400>  11
tgctattgct gctgatcgtt acnnngacaa aattgcagct ggcgttt                    47


<210>  12
<211>  47
<212>  DNA
<213>  Artificial Sequence

<220>
<223>  Primer


<220>
<221>  misc_feature
<222>  (23)..(25)
<223>  n is a, c, g, or t

<400>  12
aaacgccagc tgcaattttg tcnnngtaac gatcagcagc aatagca                    47


<210>  13
<211>  42
<212>  DNA
<213>  Artificial Sequence

<220>
<223>  Primer


<220>
<221>  misc_feature
<222>  (19)..(21)
<223>  n is a, c, g, or t

<400>  13
ggcgaaacca tcactaccnn naaactgggt ttcgttctgc tg                    42


<210>  14

<211>  42
<212>  DNA
<213>  Artificial Sequence

<220>
<223>  Primer


<220>
<221>  misc_feature
<222>  (22)..(24)
<223>  n is a, c, g, or t

<400>  14
cagcagaacg aaacccagtt tnnnggtagt gatggtttcg cc                          42


<210>  15
<211>  45
<212>  DNA
<213>  Artificial Sequence

<220>
<223>  Primer

<400>  15
ctgctggaat cgttcccgga cgcacgtgac acagaatatt tcacg                       45


<210>  16
<211>  45
<212>  DNA
<213>  Artificial Sequence

<220>
<223>  Primer

<400>  16
cgtgaaatat tctgtgtcac gtgcgtccgg gaacgattcc agcag                       45


<210>  17
<211>  47
<212>  DNA
<213>  Artificial Sequence

<220>
<223>  Primer


<220>
<221>  misc_feature
<222>  (23)..(25)
<223>  n is a, c, g, or t

<400>  17
gtcttacact gttgaaaagc tgnnngaatc gttcccggac tggcgtg                     47


<210>  18
<211>  47
<212>  DNA
<213>  Artificial Sequence

<220>
<223>  Primer

<220>
<221>  misc_feature
<222>  (23)..(25)
<223>  n is a, c, g, or t

<400>  18
cacgccagtc cgggaacgat tcnnncagct tttcaacagt gtaagac                47


<210>  19
<211>  39
<212>  DNA
<213>  Artificial Sequence

<220>
<223>  Primer


<220>
<221>  misc_feature
<222>  (19)..(21)
<223>  n is a, c, g, or t

<400>  19
gaatcgttcc cggactggnn ncgtgacaca gaatatttc                39


<210>  20
<211>  39
<212>  DNA
<213>  Artificial Sequence

<220>
<223>  Primer


<220>
<221>  misc_feature
<222>  (19)..(21)
<223>  n is a, c, g, or t

<400>  20
gaaatattct gtgtcacgnn nccagtccgg gaacgattc                39


<210>  21
<211>  45
<212>  DNA
<213>  Artificial Sequence

<220>
<223>  Primer

<400>  21
ggcgaatatg aactggcaaa aatgctgatg cgcaagggct ctctg                45


<210>  22
<211>  45
<212>  DNA
<213>  Artificial Sequence

<220>
<223>  Primer

<400>  22
cagagagccc ttgcgcatca gcatttttgc cagttcatat tcgcc                45

<210>  23
<211>  54
<212>  DNA
<213>  Artificial Sequence

<220>
<223>  Primer

<400>  23
cgtgaaaacc tgttcaccaa atgcactgat gaagaatatg aactggcaaa aatg                54


<210>  24
<211>  54
<212>  DNA
<213>  Artificial Sequence

<220>
<223>  Primer

<400>  24
catttttgcc agttcatatt cttcatcagt gcatttggtg aacaggtttt cacg                54


<210>  25
<211>  777
<212>  DNA
<213>  Manihot esculenta


<220>
<221>  CDS
<222>  (1)..(777)

<400>  25
atg gtt act gca cac ttc gtt ctg att cac acc att tgt cac ggc gca          48
Met Val Thr Ala His Phe Val Leu Ile His Thr Ile Cys His Gly Ala
1               5                   10                  15

tgg att tgg cac aaa ctg aaa ccg gcc ctg gaa cgt gct ggc cac aaa          96
Trp Ile Trp His Lys Leu Lys Pro Ala Leu Glu Arg Ala Gly His Lys
            20                  25                  30

gtt act gca ctg gac atg gca gcc agt ggc att gac ccg cgt caa att          144
Val Thr Ala Leu Asp Met Ala Ala Ser Gly Ile Asp Pro Arg Gln Ile
        35                  40                  45

gaa cag atc aac tct ttc gat gaa tac tct gaa ccg ctg ctg act ttc          192
Glu Gln Ile Asn Ser Phe Asp Glu Tyr Ser Glu Pro Leu Leu Thr Phe
    50                  55                  60

ctg gaa aaa ctg ccg caa ggc gaa aag gtt atc att gtt ggt gaa agc          240
Leu Glu Lys Leu Pro Gln Gly Glu Lys Val Ile Ile Val Gly Glu Ser
65                  70                  75                  80

tgt gca ggc ctg aac att gct att gct gct gat cgt tac gtt gac aaa          288
Cys Ala Gly Leu Asn Ile Ala Ile Ala Ala Asp Arg Tyr Val Asp Lys
                85                  90                  95

att gca gct ggc gtt ttc cac aac tcc ctg ctg ccg gac acc gtt cac          336
Ile Ala Ala Gly Val Phe His Asn Ser Leu Leu Pro Asp Thr Val His
            100                 105                 110

agc ccg tct tac act gtt gaa aag ctg ctg gaa tcg ttc ccg gac tgg          384
Ser Pro Ser Tyr Thr Val Glu Lys Leu Leu Glu Ser Phe Pro Asp Trp
        115                 120                 125

cgt gac aca gaa tat ttc acg ttc acc aac atc act ggc gaa acc atc     432
Arg Asp Thr Glu Tyr Phe Thr Phe Thr Asn Ile Thr Gly Glu Thr Ile
    130                 135                 140

act acc atg aaa ctg ggt ttc gtt ctg ctg cgt gaa aac ctg ttc acc     480
Thr Thr Met Lys Leu Gly Phe Val Leu Leu Arg Glu Asn Leu Phe Thr
145                 150                 155                 160

aaa tgc act gat gaa gaa tat gaa ctg gca aaa atg ctg atg cgc aag     528
Lys Cys Thr Asp Glu Glu Tyr Glu Leu Ala Lys Met Leu Met Arg Lys
                165                 170                 175

ggc tct ctg ttc caa aac gtt ctg gct cag cgt ccg aag ttc act gaa     576
Gly Ser Leu Phe Gln Asn Val Leu Ala Gln Arg Pro Lys Phe Thr Glu
            180                 185                 190

aaa ggc tac ggc tct atc aag aaa gtt tat att tgg acc gat caa gac     624
Lys Gly Tyr Gly Ser Ile Lys Lys Val Tyr Ile Trp Thr Asp Gln Asp
        195                 200                 205

aaa ata ttc ctg ccg gac ttc caa cgc tgg caa att gca aac tac aaa     672
Lys Ile Phe Leu Pro Asp Phe Gln Arg Trp Gln Ile Ala Asn Tyr Lys
    210                 215                 220

ccg gac aag gtt tat cag gtt caa ggc ggc gat cac aag ctg cag ctg     720
Pro Asp Lys Val Tyr Gln Val Gln Gly Gly Asp His Lys Leu Gln Leu
225                 230                 235                 240

aca aaa act gaa gaa gta gct cac att ctg caa gaa gtt gct gat gca     768
Thr Lys Thr Glu Glu Val Ala His Ile Leu Gln Glu Val Ala Asp Ala
                245                 250                 255

tac gct taa                                                         777
Tyr Ala

<210>  26
<211>  258
<212>  PRT
<213>  Manihot esculenta

<400>  26

Met Val Thr Ala His Phe Val Leu Ile His Thr Ile Cys His Gly Ala
1               5                   10                  15

Trp Ile Trp His Lys Leu Lys Pro Ala Leu Glu Arg Ala Gly His Lys
            20                  25                  30

Val Thr Ala Leu Asp Met Ala Ala Ser Gly Ile Asp Pro Arg Gln Ile
        35                  40                  45

Glu Gln Ile Asn Ser Phe Asp Glu Tyr Ser Glu Pro Leu Leu Thr Phe
    50                  55                  60

Leu Glu Lys Leu Pro Gln Gly Glu Lys Val Ile Ile Val Gly Glu Ser
65                  70                  75                  80

Cys Ala Gly Leu Asn Ile Ala Ile Ala Ala Asp Arg Tyr Val Asp Lys
                85                  90                  95

Ile Ala Ala Gly Val Phe His Asn Ser Leu Leu Pro Asp Thr Val His
            100                 105                 110

Ser Pro Ser Tyr Thr Val Glu Lys Leu Leu Glu Ser Phe Pro Asp Trp
        115                 120                 125

Arg Asp Thr Glu Tyr Phe Thr Phe Thr Asn Ile Thr Gly Glu Thr Ile
    130                 135                 140

Thr Thr Met Lys Leu Gly Phe Val Leu Leu Arg Glu Asn Leu Phe Thr
145                 150                 155                 160

Lys Cys Thr Asp Glu Glu Tyr Glu Leu Ala Lys Met Leu Met Arg Lys
                165                 170                 175

Gly Ser Leu Phe Gln Asn Val Leu Ala Gln Arg Pro Lys Phe Thr Glu
            180                 185                 190

Lys Gly Tyr Gly Ser Ile Lys Lys Val Tyr Ile Trp Thr Asp Gln Asp
        195                 200                 205

Lys Ile Phe Leu Pro Asp Phe Gln Arg Trp Gln Ile Ala Asn Tyr Lys
    210                 215                 220

Pro Asp Lys Val Tyr Gln Val Gln Gly Gly Asp His Lys Leu Gln Leu
225                 230                 235                 240

Thr Lys Thr Glu Glu Val Ala His Ile Leu Gln Glu Val Ala Asp Ala
                245                 250                 255

Tyr Ala